# EUROPEAN PATENT APPLICATION

(11) **EP 4 379 734 A1**
(43) Date of publication of application: **05.06.2024**
(21) Application number: 22210672.6
(22) Date of filing: 30.11.2022
(51) Int. Cl.: G16H 20/40, A61B 5/0205, A61B 5/00, G06F 3/0484, G06T 11/20, G16H 40/60

(54) **SYSTEM AND METHOD FOR PATIENT MONITORING**

(71) Applicant: Koninklijke Philips N.V., 5656 AG Eindhoven (NL)
(72) Inventor: SARTOR, Francesco, 5656AG Eindhoven (NL); HUIJBREGTS, Laurentia Johanna, 5656AG Eindhoven (NL); BULUT, Murtaza, 5656AG Eindhoven (NL); LOPEZ LOPEZ, Benjamin, 5656AG Eindhoven (NL); DANISMAN TASAR, Mine, 5656AG Eindhoven (NL); BERA, Deep, 5656AG Eindhoven (NL); VAN LIESHOUT, Ron Martinus Laurentius, 5656AG Eindhoven (NL)
(74) Representative: Philips Intellectual Property & Standards

(57) **Abstract**

A system and computer-implemented method may be provided for patient monitoring. The system and method may establish a graphical user interface on-screen which comprises a trend view 200. The trend view may provide a longitudinal visualization setting out measured values of physiological parameters at consecutive time instances against a common timeline 210. When user input is received which represents a selection of a first point 240 in the trend view, a first time instance on the common timeline may be determined and a time window 260, 262 on the common timeline may be selected based on the first time instance. A numerical visualization 230-234 may then be generated of the measured values of one or more of the physiological parameters at respective endpoints of the time window, enabling the user to easily see the changes in the physiological parameter(s) across time.

## Description

### FIELD OF THE INVENTION

The invention relates to a system and computer-implemented method for patient monitoring. The invention further relates to a computer-readable medium comprising instructions for a computer program, the computer program comprising instructions to cause a processor system to perform the computer-implemented method.

### BACKGROUND OF THE INVENTION

It is known to monitor physiological parameters of a subject at regular and consecutive time instances. For example, the heartrate of a runner may be monitored to serve as a performance metric during a run. If the monitoring is in the context of medical care, the subject may also be referred to as patient and such monitoring as patient monitoring. In both cases, the measured values of the physiological parameters may be visualized, for example for the user or patient to see (e.g., in self-care scenarios) or for medical personnel too see. Typically, in patient monitoring, the measured values may be compared to static or dynamic thresholds so detect abnormalities in the physical parameters, and if such abnormalities are detected, an alarm may be triggered, e.g., to attract the attention of medical personnel. For example, on an intensive care unit (ICU), a patient's vital signs (heart rate (HR), respiration rate (RR), core body temperature (CBT), oxygen saturation (SpO2) and blood pressure (BP)) may be closely monitored. In general, physiological parameters may include, but are not limited to, vital signs such as heart rate, respiration rate, and blood pressure, advanced hemodynamic parameters such as cardiac output, stroke volume, and systemic vascular resistance, haematological parameters, such as red and white blood cell counts and haemoglobin concentration, and analyte parameters, such as glucose or lactate, concentrations in whole blood, plasma, or serum.

It is known in patient monitoring to show a trend view of the measured values of the physiological parameters on a display. Such a trend view may provide a longitudinal visualization of the measurement data by setting out the measured values of the physiological parameters at the consecutive time instances against a common timeline. Typically, in such a trend view, the time is presented on the x-axis while the measured values of the physiological parameters are presented on the y-axis, typically in the form of graphs which are vertically separated from each other along the y-axis but horizontally aligned with respect to the time on the x-axis. Based on the trend view, medical personnel may spot trends in the measured physiological parameters, such as a deterioration in the medical condition of the patient, and intervene in the patient's treatment, if necessary.

It is known to equip a patient monitor with a user interface that enables a user, such as medical personnel, to better perceive and understand the information presented by the patient monitor. For example, US2008076977A1 describes a patient monitor with a user interface that allows a user to obtain a snapshot view, with the term 'snapshot view' referring to information on a display that is normally changing in real-time operation, but which is held fixed in the snapshot view for a period of time which is sufficient for a user to more readily interpret, comprehend, or otherwise understand its content.

While this snapshot view may contribute to better perceiving and understanding some information presented by the patient monitor, further improvements are desirable. In particular, it has been found that it is difficult to perceive and understand changes in the measured values over time from the graphs presented in a trend view.

### SUMMARY OF THE INVENTION

In a first aspect of the invention, a system is provided for patient monitoring, comprising:
an input interface for receiving measurement data comprising measured values of physiological parameters of a patient at consecutive time instances;
a user interface subsystem comprising:
   - a user input interface to a user input device for receiving user input;
   - a display output to a display for displaying output of the system;
a processor subsystem configured to, via the user interface subsystem:
   - establish a graphical user interface on the display, wherein the graphical user interface comprises a trend view which provides a longitudinal visualization of the measurement data by setting out the measured values of the physiological parameters at the consecutive time instances against a common timeline;
   - receive user input representing a selection of a first point in the trend view, wherein the first point corresponds to a first time instance on the common timeline;
   - select a time window on the common timeline based on the first time instance; and
   - generate a numerical visualization of the measured values of one or more of the physiological parameters at respective endpoints of the time window.

In a further aspect of the invention, a computer-implemented method is provided for patient monitoring, comprising:
receiving measurement data comprising measured values of physiological parameters of a patient at consecutive time instances;
establishing a graphical user interface on a display, wherein the graphical user interface comprises a trend view which provides a longitudinal visualization of the measurement data by setting out the measured values of the physiological parameters at the consecutive time instances against a common timeline;
receiving user input representing a selection of a first point in the trend view, wherein the first point corresponds to a first time instance on the common timeline;
selecting a time window on the common timeline based on the first time instance; and
generating a numerical visualization of the measured values of one or more of the physiological parameters at respective endpoints of the time window.

In a further aspect of the invention, a transitory or non-transitory computer-readable medium is provided, the computer-readable medium comprising data representing a computer program comprising instructions for causing a processor system to perform a computer-implemented method as described in this specification.

The above measures relate to patient monitoring, which may typically involve monitoring the condition of a patient in a clinical setting, e.g., in a hospital, but which may also include non-clinical settings, e.g., at home in a self-care setting, as well as non-medical settings, e.g., when monitoring the performance of a sporter. In such patient monitoring, physiological parameters of the patient may be measured at consecutive time instances, for example at regular intervals, e.g., every 100ms, 1 sec, 1 minute, 10 minutes, 1 hour, etc., or at irregular intervals, e.g., when changes in the physiological parameters are expected to occur. For such measurements, various known types of sensors and/or sensing devices may be used, including but not limited to the types described elsewhere in this specification.

The above measures involve providing a system which has access to the measured values of physiological parameters of a patient. For example, the system may be a patient monitor which comprises or is directly connected to the aforementioned sensors and/or sensing devices. Another example is that the system may be a workstation or mobile device which provides a user with remote access to the measured values of the physiological parameters measured by such a patient monitor. The system comprises a user interface subsystem via which the system may present a graphical user interface on-screen and via which the system may receive input from the user. The graphical user interface may comprise a trend view which provides a longitudinal visualization of the measurement data. In particular, the trend view may set out the measured values of the physiological parameters at the consecutive time instances against a common timeline. Typically, the common timeline may be presented set out on one axis, e.g., the x-axis, while the measured values of the physiological parameters may be set out on the other axis, e.g., the y-axis, for example in separate graphs which are spaced apart along the other axis. It is noted that the functionality of the system described in this paragraph may be known per se. It is further noted that in addition to displaying physiological parameters, the trend view may also include visualizations of events, such as interventions, and visualizations of bookmarks.

The above measures may further involve enabling a user to easily obtain a numerical visualization of the measured values of one or more of the physiological parameters at two time instances along the common timeline. Such a numerical visualization may involve displaying a respective measurement value numerically, e.g., as a number, such as "98%" for blood saturation, "60" for heart rate, etc. Since the common timeline extends from a current time back into the past, typically at least one of these time instances may lie in the past. The time instances may together define a time window along the common timeline wherein both time instances represent the respective endpoints of the time window. The time window is thus considered to be represented by the two time instances, while conversely the two time instances are jointly considered to define the time window.

The user may select the time window. This may involve the user selecting a point in the trend view. For example, if the display on which the trend view is presented is a touch screen, the user may select the point by pressing somewhere within the trend view. Another example is that a point may be selected via a mouse cursor or the like. The selected point may then be mapped by the system to a time instance on the common timeline. Since the common timeline may correspond to one of the axes of the trend view, such a mapping may be performed by, or at least understood as, an orthogonal projection of the selected point onto said axis. An exemplary implementation of such a mapping may involve determining the coordinate of the selected point in a coordinate system associated with the trend view and considering the coordinate of the selected point in the time dimension to represent the time instance. The thus selected time instance may then be used to determine a time window on the common timeline. For example, the time instance may be used as an intermediate point or as an endpoint of the time window, thereby at least in part defining the time window. Another example is that the time instance may be nearby in time to an event which visualized onscreen in relation to the common timeline, and the selection of the time instance may cause the above measures to select the time window to correspond to this nearby event. Yet another example is that the time instance may be within or nearby in time to a previously bookmarked time window and the selection of the time instance by the user may cause the above measures to select this previously bookmarked time window.

Having selected the time window, a numerical visualization of the measured values of physiological parameters at both ends of the time window may be provided. This may involve, for a respective physiological parameter, simultaneously displaying the numerical value of the physiological parameter at the first time instance and at the second time instance. Such simultaneous numerical visualizations may be provided for at least a subset of the physiological parameters which are viewable in the trend view. For example, the numerical value(s) pertaining to the first time instance may be displayed within the trend view in visual correspondence with the first time instance while the numerical value(s) pertaining to the second time instance may be displayed in visual correspondence with the second time instance. Here, the term 'visual correspondence' may for example comprise displaying the numerical values aligned with or in a neighbourhood of the respective time instance so that a user may determine that the displayed numerical values pertain to the particular time instance.

The above measures may have the advantage that a user may easily obtain an overview of the changes of one or more physiological parameters over time as the user is provided with a simultaneous display of the numerical values at a first time instance and at a second time instance, thereby enabling the user to easily see the changes in the physiological parameter between both time instances. While it may be possible for a user to see such changes from graphs presented in the trend view, the graphs may be difficult to read accurately, particularly when seeking to compare two time instances which are spaced far apart. By providing a numerical visualization for the respective time instances, such interpretation issues may be alleviated. At the same time, it may be relatively easy for the user to obtain such an overview, as the user may select the time instance by selecting a point in the trend view and without having to precisely select a point on the visualization of the common timeline. Rather, the user may select a point substantially anywhere within the trend view, and the system may then map the point to a time instance on the common timeline. This may allow the user to focus only on being accurate in one dimension, e.g., along the time axis, without requiring the user to also be accurate in the other, typically orthogonal, dimension.

Optionally, the processor subsystem is configured to receive further user input representing a selection in the trend view and to determine a width of the time window based on the further user input. In addition to selecting a time instance on the common timeline, the user may also provide further user input by which a width of the time window may be determined. This way, the user may not only control the placement of the time window in time but also the width of the time window, e.g., its start and end.

Optionally, the further user input comprises the selection of a second point in the trend view, wherein the second point corresponds to a second time instance on the common timeline, and wherein the processor subsystem is configured to use the first time instance as a first endpoint of the time window and the second time instance as a second endpoint of the time window. As further user input, the user may select a second time instance on the common timeline by way of selecting a second point in the trend view. This way, the user may select both time instances in a relatively direct manner, while the user may indirectly determine the width of the time window, namely as the distance between both selected time instances. A relatively direct selection of time instances may be advantageous if the user wishes to obtain a numerical visualization of the measured values of physiological parameter(s) at known time instances. Advantageously, the system may be configured such that an initial selection of a point determines the first time instance, and a subsequent selection of a point determines the second time instance. This may allow a user to select both time instances in a straightforward manner, for example using a touch screen, and without requiring more complex user interactions, such as holding down a mouse button or the like, which may be cumbersome and prone to errors in daily clinical practice.

Optionally, the further user input comprises a continued or repeated selection of the first point, wherein the processor subsystem is configured to use the first time instance as the intermediate point, for example a midpoint, of the time window and to determine the width of the time window based on a time length of the continued selection, or based on a number of the repeated selection, of the first point. The user may know the approximate midpoint of the time window for which he/she wishes to visualize the numerical values of measured physiological parameter(s). An intuitive user interface may be provided in which the user may select the midpoint of the time window and in which the system determines the width of the time window based on how long, or how often, the user selects the midpoint. For example, the user may select a point in the trend view shown on a touch screen using his/her finger and continue to hold his/her finger on-screen, which may cause the system to establish and visualize a time window centred around the selected point and to gradually increase the width of the time window until the user lifts his/her finger off the screen, at which point the width of the time window may remain fixed. Alternatively, how often a user repeatedly selects the point in the trend view may determine the width of the time window. For example, a first selection of a point may result in a time window of a certain width, e.g., 1x, while a repeated second selection of the point may result in a time window of width 2x, etc. Such a user interface may further facilitate the user's selection of a time window.

Optionally, the trend view further provides a visualization of an event with respect to the common timeline, wherein the event has a start time and an end time on the common timeline, wherein the selection of the first point is a selection of or within the visualization of the event, and wherein the processor subsystem is configured to select the time window to include the event. There may be events associated with the patient monitoring which may be logged or otherwise known to the system. For example, a patient may be treated, e.g., with intravenous drugs, and the administration of the drugs may be registered by the system, e.g., in form of a starting time and an ending time. The trend view may visualize such events in relation to the common timeline. A user may wish to overview of the changes of one or more physiological parameters over time before and after the event. For that purpose, the system may enable the user to select the visualization of the event on-screen, and the time window may be determined to include the event. For example, the time window may be set to exactly match the event, or to include the event and some time before and after the event. This way, the user may easily to see the changes in the physiological parameter(s) before and after the event. This may be of particular relevance in daily clinical practice as it may allow medical personnel to determine the efficacy of treatment, to monitor for adverse effects, etc.

Optionally, the processor subsystem is configured to use the start time of the event as a first endpoint of the time window and the end time of the event as a second endpoint of the time window.

Optionally, the event is associated with a subset of the physiological parameters of the patient, wherein the processor subsystem is configured to generate the numerical visualization of the measured values selectively for the subset of the physiological parameters. Depending on the type of event, only a subset of physiological parameters may be of particular relevance, for example as a particular type of event may primarily affect certain physiological parameters. To allow a user to focus on these physiological parameters, the system may generate the numerical visualization of the measured values selectively (e.g., only) for the subset of the physiological parameters.

Optionally, wherein the processor subsystem is configured to highlight the measured values of the subset of the physiological parameters in the trend view and/or omit or deemphasize the measured values of physiological parameters which are not part of the subset. Measured values of physiological parameters which are not or of lesser relevance for the event may be hidden or deemphasized by the system. This way, the user may be focus on the physiological parameters of relevance without distraction.

Optionally, the processor subsystem is configured to enable a user to select a next or previous event on the common timeline and, in response to a selection of the next or previous event by the user, adapt the time window to the selection of the next or previous event. Once the user has selected an event, a next or previous event may be selected, for example by pressing 'left' and 'right' keys or corresponding arrows in the graphical user interface and without requiring a user to select the respective events directly via touch or a mouse cursor. This may allow a user to effectively 'scroll' through the events to obtain an overview of past and current events and to determine their effect on the physiological parameters based on the visualized numerical values.

Optionally, the event represents or comprises a medical intervention with respect to the patient.

Optionally, the processor subsystem is configured to adjust a scale of the common timeline to fit the time window.

Optionally, the processor subsystem is configured to determine a numerical difference between the measured values of the one or more of the physiological parameters at the respective endpoints of the time window and to visualize the numerical difference. In addition to visualizing the absolute values of the measured physiological parameters, also the relative changes may be visualized. This may allow a user to quickly determine the change in physiological parameters across time.

Optionally, visualizing the numerical difference comprises visualizing a sign and/or magnitude of the numerical difference. For example, an increase in heart rate from 80bpm to 85bpm may be visualized as "+5" and a decrease as "-5".

It will be appreciated by those skilled in the art that two or more of the above-mentioned embodiments, implementations, and/or optional aspects of the invention may be combined in any way deemed useful.

Modifications and variations of the system, the computer-implemented method and/or the computer program product, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which:
Fig. 1 shows a system for patient monitoring, wherein the system is configured to display a graphical user interface, wherein the graphical user interface comprises a trend view showing measured values of physiological parameters over time;
Fig. 2A shows an example of a trend view;
Fig. 2B shows the selection of a first point in the trend view, wherein the first point corresponds to a first time instance on the time axis;
Fig. 2C shows a numerical display of measured values of the physiological parameters at the first time instance, wherein a second point is selected in the trend view, wherein the second point corresponds to a second time instance on the time axis, and wherein the first time instance and the second time instance together define a time window;
Fig. 2D shows a numerical display of measured values of the physiological parameters at both endpoints of the time window;
Fig. 3A shows a continued selection of a point in the trend view, wherein the point corresponds to a time instance on the time axis, wherein the selection of the point causes a time window to be established which is centred around the point, and wherein the time of continued selection of the point determines a width of the time window;
Fig. 3B shows the time window obtained by the continued selection of the point, wherein the trend view comprises a numerical display of the measured values of the physiological parameters at both endpoints of the time window;
Fig. 4A shows a selection of a visualization of an intervention, wherein the intervention has a start time and an end time which together define a time window;
Fig. 4B shows the time window obtained by the selection of the visualization of the intervention, wherein the trend view comprises a numerical display of the measured values of the physiological parameters at both endpoints of the time window;
Fig. 5 illustrates a hiding or deemphasizing of graphs of physiological parameters which are of no or lesser relevance for the selected intervention;
Fig. 6 shows the selection of a previous intervention;
Fig. 7 shows a method for patient monitoring; and
Fig. 8 shows a non-transitory computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 20: data storage
- 40: physiological signals
- 42: measurement data
- 60: display
- 62: display data
- 80: user input device
- 82: user input data

- 100: system for patient monitoring
- 110: data storage interface
- 120: sensor interface
- 140: processor subsystem
- 142-148: data communication
- 160: memory
- 180: user interface subsystem
- 182: display output interface
- 184: user input interface

- 200: trend view
- 210: time axis
- 212: graph of measured values of physiological parameter
- 214: hidden graph of measured values of physiological parameter
- 220,222: intervention
- 230-234: numerical visualization of measured value
- 240: selection of first point
- 242: selection of second point
- 244: continued selection of point
- 246: selection of (first) point
- 250: orthogonal projection onto time axis
- 260, 262: time window

- 300: method for patient monitoring
- 310: receiving measurement data
- 320: establishing graphical user interface on display
- 330: receiving user input
- 340: selecting time window
- 350: displaying measured values at endpoints of time window

- 400: non-transitory computer-readable medium
- 410: data representing computer program

### DETAILED DESCRIPTION OF EMBODIMENTS

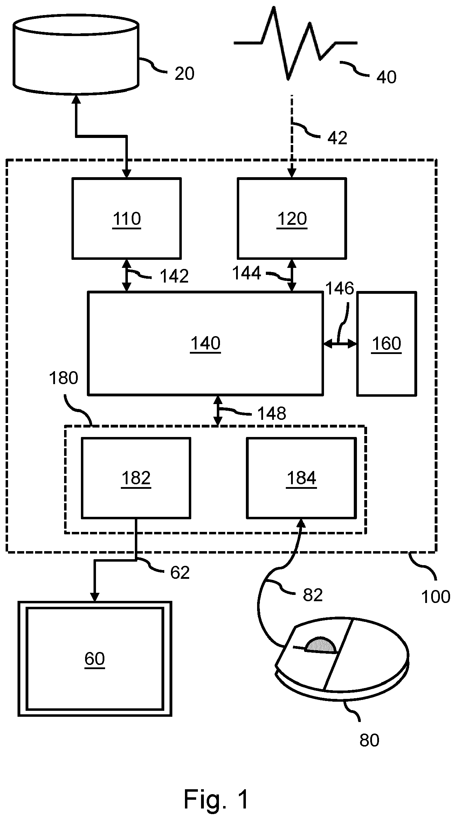

**Fig. 1** shows a system 100 for patient monitoring. The system 100 may comprise a sensor interface 120 for obtaining measurement data 42 representing measurement values of physiological signals 40. Such measurement data 42 may for example be obtained from a sensor or from a sensing device, or a combination of such sensors and/or sensing devices. Examples of sensors and sensing devices include, but are not limited to, a Swan Ganz pulmonary artery catheter (PAC), Philips PiCCO (which combines an arterial line and a central venous line), Edwards ClearSight (which uses finger cuffs), Edwards FloTrac (using an arterial line), echography, Philips ShellCuff (using a cuff around the upper arm), etc. The measurement data may for example be received in real-time or near real-time. Effectively, the sensor interface 120 may constitute an input interface for the measurement data 42 and may elsewhere also be referred to as an input interface.

The system 100 may further comprise a data storage interface 110 to a data storage 20. The data storage 20 may serve as short term and/or long-term data storage. For example, the measurement data 42 obtained via the sensor interface 120 may be at least temporarily stored on the data storage 20. In some embodiments, the measurement data 42 may also be accessed from the data storage 20 instead of using a sensor interface, for example in cases when the measurement data 42 is pre-recorded measurement data or when the measurement data 42 represents simulated measurement data. In the example of Fig. 1, the data storage interface 110 is shown to be connected to an external data storage 20. Alternatively, the data storage 20 may be an internal data storage of the system 100 (not shown in Fig. 1). In general, the data storage interface 110 may take various forms, such as a hard disk or solid-state disk interface to one or more hard disks and/or solid state disks, or a network interface to a Local Area Network (LAN) or a Wide Area Network (WAN).

The system 100 is further shown to comprise a processor subsystem 140 configured to internally communicate with the sensor interface 120 via data communication 144, with the data storage interface 110 via data communication 142, with a memory 160 via data communication 146 and with a user interface subsystem 180 via data communication 148. The memory 160 may for example be a volatile memory in which a computer program may be loaded which may cause the processor subsystem 140 to carry out functions which are described in this specification as being performed by the processor subsystem.

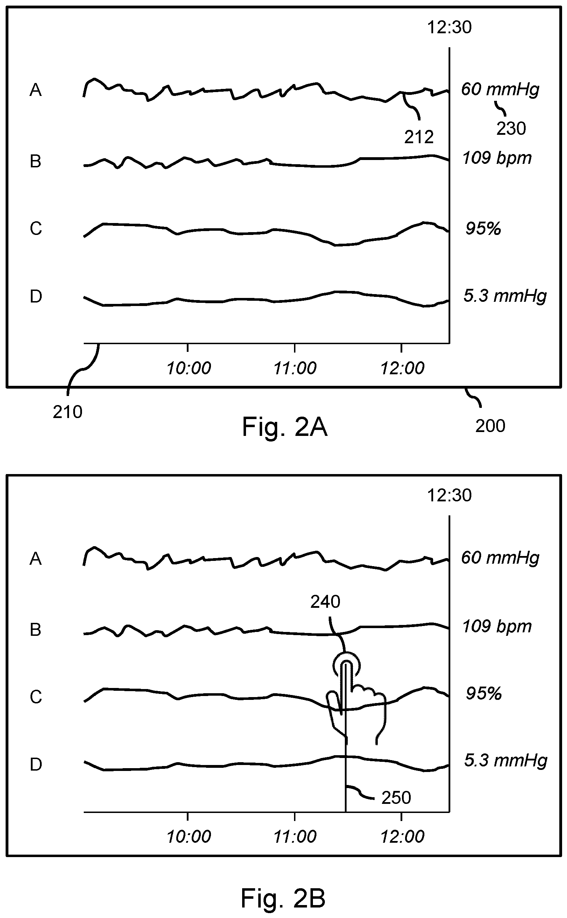

The user interface subsystem 180 may be configured to, during operation of the system 100, enable a user to interact with the system 100, for example using a graphical user interface. In particular, as also described with reference to Fig. 2A et seq., the graphical user interface may enable the user to select a point in a trend view of a graphical user interface. For that and other purposes, the user interface subsystem 180 is shown to comprise a user input interface 184 configured to receive user input data 82 from a user input device 80 operable by the user. The user input device 80 may take various forms, including but not limited to a computer mouse, touch screen, keyboard, microphone, etc. Fig. 1 shows the user input device to be a computer mouse 80. In general, the user input interface 184 may be of a type which corresponds to the type of user input device 80, i.e., it may be a thereto corresponding type of user device interface. The user interface subsystem 180 is further shown to comprise a display output interface 182 configured to provide display data 62 to a display 60 to visualize output of the system 100. In the example of Fig. 1, the display is an external display 60. Alternatively, the display may be an internal display.

As also described with reference to Fig. 2A et seq., the processor subsystem 140 may be configured to, during operation of the system 100 and using the user interface subsystem 180, establish a graphical user interface on the display, wherein the graphical user interface comprises a trend view which provides a longitudinal visualization of the measurement data by setting out the measured values of the physiological parameters at the consecutive time instances against a common timeline. The processor subsystem 140 may be further configured to receive user input representing a selection of a first point in the trend view, wherein the first point corresponds to a first time instance on the common timeline, select a time window on the common timeline based on the first time instance, and generate a numerical visualization of the measured values of one or more of the physiological parameters at respective endpoints of the time window. These and other operations of the system 100, and various optional aspects thereof, will be explained in more detail with reference to Fig. 2A et seq.

In general, the system 100 may be embodied as, or in, a single device or apparatus. The device or apparatus may be a general-purpose device or apparatus, such as a workstation or a computer, but may also be application-specific, such as a patient monitor. The device or apparatus may comprise one or more microprocessors which may represent the processor subsystem, and which may which execute appropriate software. The software may have been downloaded and/or stored in a corresponding memory, e.g., a volatile memory such as RAM or a non-volatile memory such as Flash. Alternatively, the functional units of the system, e.g., the input interface, the user interface subsystem, and the processor subsystem, may be implemented in the device or apparatus in the form of programmable logic, e.g., as a Field-Programmable Gate Array (FPGA). In general, each functional unit of the system 100 may be implemented in the form of a circuit. It is noted that the system 100 may also be implemented in a distributed manner, e.g., involving different devices or apparatuses. For example, the distribution may be in accordance with a client-server model, e.g., using a server and workstation. For example, the user input interface and the display output interface may be part of the workstation, while the processor subsystem may be a subsystem of the server. It is noted that various other distributions are equally conceivable.

**Fig. 2A** shows an example of a trend view 200 which may be displayed by the system 100 of Fig. 1. In this example, the trend view 200 is shown to comprise a horizontal axis 210 along which time is set out and which axis may also be referred to as time axis, and a vertical axis along which the measured values of several physiological parameters are set out, being in this example from top to bottom the mean arterial pressure, the heart rate, blood oxygen saturation and the central venous pressure. In the example of Fig. 2A and elsewhere, the measured values of the physiological parameters are shown in separate graphs 212 which are spaced apart along the vertical axis, but this is not a limitation, as it may also be possible to overlay such graphs, e.g., where physiological parameters are expressed in a same unit, or to show them in any other suitable manner. Typically, during operation of the system, the current or most recent measured values of the physiological parameters are plotted at the right-hand side of the trend view 200 while past measured values are over time moved toward the left-hand side of the trend view before falling outside of the time range of the trend view. Notably, the measured values of the physiological parameters in the individual graphs are mutually aligned with respect to the time axis 210 and therefore aligned with respect to a common timeline as represented by the time axis. In addition, the current or most recent measured values of the physiological parameters are numerically visualized 230 at the right-hand side of the trend view, showing that the current or most recent measured values are respectively "60 mmHg", "109 bpm", "95%", and "5.3 mmHg". The trend view 200 may thus provide a longitudinal visualization of the measurement data by setting out the measured values of the physiological parameters at the consecutive time instances against a common timeline.

**Fig. 2B** illustrates functionality of the system 100 of Fig. 1 by which a user may select a point 240 in the trend view so as to select a time instance on the common timeline. In the example of Fig. 2B and elsewhere, the selection by the user is carried out via touch screen, in that the user may press with his/her finger on a touch screen and thereby select a point in the trend view. It will be appreciated, however, that various other types of user input and user input devices may be used by the user to select a point in the trend view, such as for example a mouse by which the user may control an onscreen pointer. With continued reference to the selected point 240, the following is noted. In a particular input mode of the system 100 of Fig. 1, the selection of a point in the trend view may cause system to determine a time instance on the common timeline which corresponds to that point. This may be further explained as follows. The system may allow the user to select a time instance on the common timeline in an intuitive and easy-to-use manner. It is typically difficult to accurately select a point in two dimensions as the user will need to accurately position his/her finger (or mouse pointer, etc) in both dimensions simultaneously. When applying this to the selection of a time instance on the common timeline, this may mean that the user has to be vertically accurate to select the time axis and horizontally accurate to select the correct time instance on the time axis. In the aforementioned input mode, the vertical position of the user input, meaning the vertical coordinate of the selected point, may be ignored and rather only the horizontal position of the user input, meaning the horizontal coordinate of the selected point, may be further processed and considered to represent a selection of a time instance on the common timeline. Effectively, a selection of a point 240 may be considered to be orthogonally projected 250 by the system onto the common timeline. This way, the user may not need to be accurate in both dimensions of the trend view simultaneously, but may rather focus on being accurate in the time dimension.

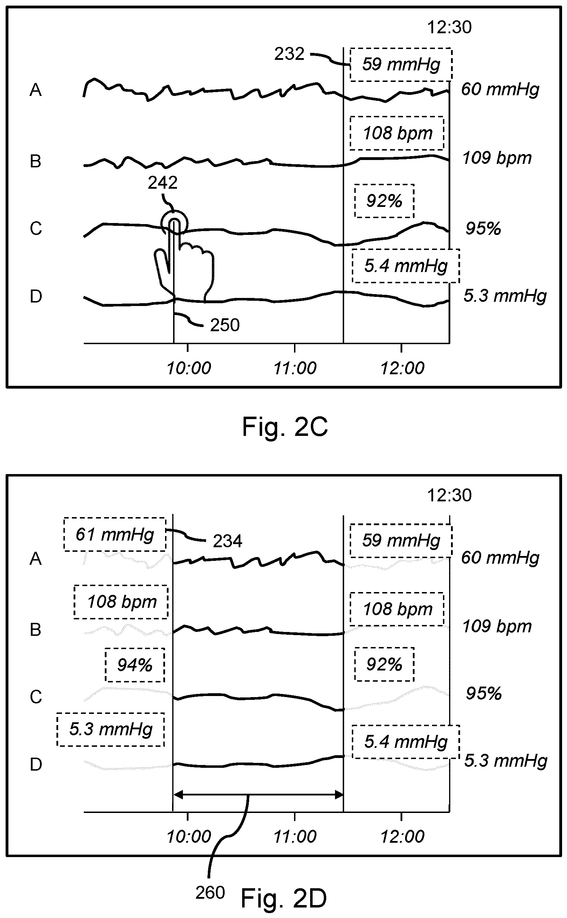

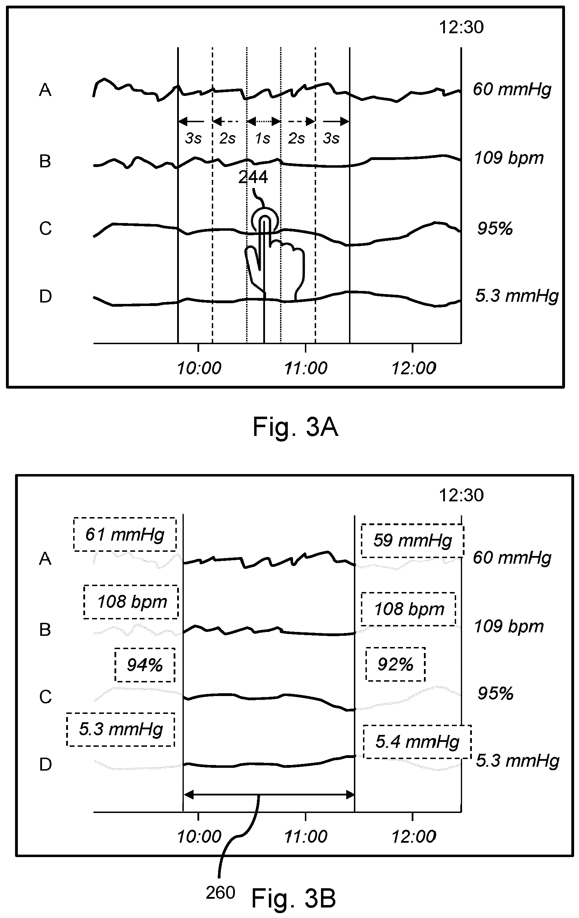

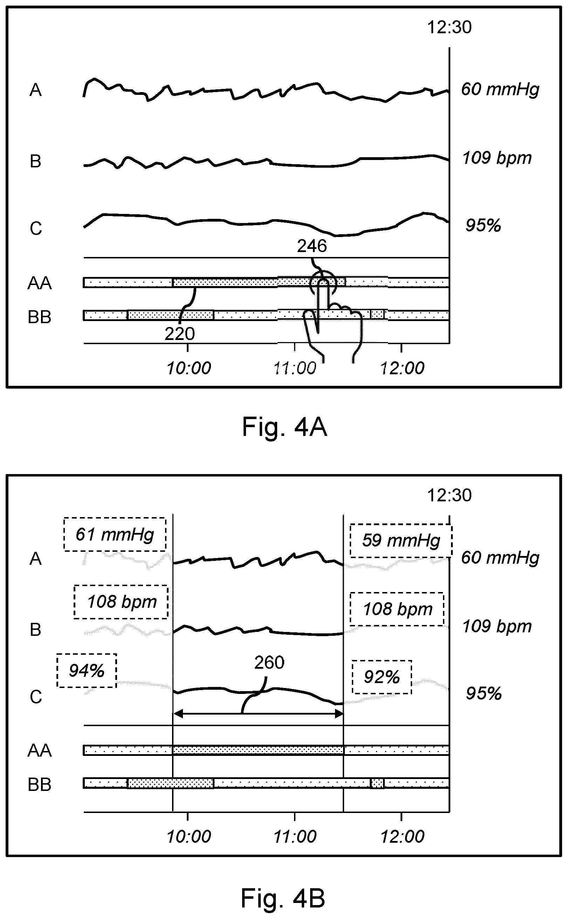

Based on the time instance selected by the user, the system 100 of Fig. 1 may select a time window on the common timeline which comprises the time instance as an intermediate point or as an endpoint. In some embodiments, further user input may be provided so as to select the time window. Examples of such further user input are described with reference to Fig. 2C, in which the further user input pertains to a selection of a second point in the trend view to select a second time instance, and with reference to Fig. 3A, in which the further user input pertains to a continued or repeated selection of the first point. However, such further user input may not be required. For example, as also described with reference to Fig. 4A, if the point selected by the user pertains to a visualization of an event in the trend view, e.g., if the user selects the event onscreen, the time window may be selected to include the event, e.g., its start and end. Another example is that the system may automatically establish a time window based on the selected time instance, for example having a default width or a width which is adaptively chosen by the system, e.g., based on the measured values of the physiological parameters near the selected time instance.

**Fig. 2C** shows a result of the selection of the point 240 in Fig. 2B, in that the system 100 of Fig. 1 may provide a numerical display 232 of measured values of the physiological parameters at the first time instance, being in this example "59 mmHg", "108 bpm", "92%", and "5.4 mmHg". The user may further select a second point 242 in the trend view, for example simultaneously with the first point 240 or consecutively. The second point 240 may be mapped by the system to a second time instance on the time axis, e.g., by orthogonal projection 250. As shown in **Fig. 2D****,** both selected time instances may be considered by the system to define a time window 260. After selection of the second point, the system may furthermore provide a numerical display 234 of measured values of the physiological parameters at the second time instance, being in this example "61 mmHg", "108 bpm", "94%", and "5.3 mmHg". A user may thus easily see the changes in the physiological parameters between both time instances by comparing the numerical values at either end of the time window 260. The example of Fig. 2D further shows that the system may horizontally align the numerical values with the respective time instance and may vertically align the numerical values with the respective physiological parameter. However, this is not a limitation, in that the numerical values may also be differently displayed, e.g., using a different layout. Also shown in Fig. 2D is that the system may hide or deemphasize measured values of the physiological parameters which fall outside of the time window 260. In the example of Fig. 2D, such deemphasizing is carried out contrast reduction but any other alternative technique for such deemphasizing may be used as well. It is noted that measured values of the physiological parameters which fall outside of the time window 260 may also be hidden by the time scale of the time axis being adjusted by the system so as to correspond to the time window 260, thereby omitting the measured values of the physiological parameters outside of the time window 260 from the trend view.

Although not shown in Fig. 2D, the system may additionally determine a numerical difference between the measured values of the one or more of the physiological parameters at the respective endpoints of the time window and visualize the numerical difference, for example by visualizing a sign and/or magnitude of the numerical difference. In the example of Fig. 2D, this may for example comprise displaying, at the righthand side of the trend view, "(-2)" behind "59 mmHg", "(0)" behind "108 bpm", "(-2)" behind 92% and "(+0.1)" behind "5.4 mmHg" to denote the changes in the respective physiological parameters between both time instances. Alternatively, such relative changes may be visualized by arrows (e.g., upward arrow to denote an increase in the measured values of a physiological parameter, downward arrow to denote a decrease), by colour coding or in any other way.

**Fig. 3A** shows an alternative to the input mechanism as described with reference to Figs. 2A-2D to select a time window. Here, the user may select a point 244 in the trend view so as to select a time instance and then continue to select the point, e.g., by continuing to press his/her finger onto a touch screen, so as to determine a width of a time window which is centred around the selected time instance. Namely, the system 100 of Fig. 1 may be configured to establish the time window after selection of the point 244 and determine the width of the time window based on the time of continued selection of the point 244. For example, a continued selection of Is may result in a time window which is 10 min. wide, while 2s may result in a time window of 20 min. wide, etc. In some embodiments, a continued selection for a minimum duration, for example 1 sec., may be required for the system to establish a time window. This way, a spurious selection of a point in the trend view may not trigger the establishment of a time window. Typically, the time window may be established symmetrically around the selected time instance. However, this is not a limitation, in that the time window may also be established asymmetrically. It is noted that, as an alternative to the continued selection of the point 244, a repeated selection of the point 244 may cause the system to determine the width of the time based on how many times the point is selected, for example 10 min. for 1x, 20 min. for 2x, 30 min. for 3x, etc.

**Fig. 3B** shows that the result of the selection of a time window using the input mechanism described with reference to Fig. 3A may be similar to that obtained using the input mechanism described with reference to Figs. 2A-2D, since both examples result in the selection of a same time window 260 which is shown both in Fig. 2D and in Fig. 3B.

With continued reference to Figs. 2A-3B, it is noted that the system 100 of Fig. 1 may be configured to provide several input mechanisms simultaneously. For example, in case of a touch screen, a selection of a point onscreen may cause the system to select a time instance, a touch and hold may cause the system to select a time window, and two simultaneous or consecutive touches may cause the system to also select a time window. In other words, the functionality illustrated in respectively Figs. 2A-2D and 3A-3B may be simultaneously supported by the system. Thus, the user may have several ways to define a time window. Yet another way to define a time window is described in the following, and also this input mechanism may be provided simultaneously with other input mechanisms.

**Fig. 4A** shows that the trend view may comprise, in addition to a visualization of the measured values of physiological parameters, also a visualization of one or more events. The events may be visualized in visual alignment with the common timeline, in that an event 220 may have a start time and an end time which horizontally match the respective times on the common timeline. In a particular input mode of the system 100 of Fig. 1, the selection of a point in the trend view which corresponds to, or lies within, a visualization of an event may cause the system to select the time window to include the event. In other words, if the user selects the event 220 by selecting a point 246 which lies within the visualization of the event (e.g., within the graphical shape visually representing the event), the system may determine the time window to include the event, for example by determining the time window to match the event (e.g., in terms of start and endpoint) or by determining the time window to be (slightly) larger than the duration of the event. As shown in **Fig. 4B****,** a result of the selection of an event may be similar as if the time window 260 were determined using the input mechanisms previously described with reference to Figs. 2A-2D and 3A-3B.

With continued reference to Fig. 4B, it is noted that the time window may also be selected to include a time instance which is deemed of particular relevance for the event. For example, in case of an intervention, the time window may be chosen to include, or end at, a time instance at which the effect of the intervention is assumed to be at a maximum (e.g., 1 min. after the end of the fluid infusion for a fluid challenge). In some cases, the end of the medical intervention may be determined from the measured values of the physiological parameters themselves. For example, an inotropic drug is known to alter the heart function. Accordingly, the heart rate and its variations may be used to locate the end of the intervention and the time window may be set accordingly to end at the intervention.

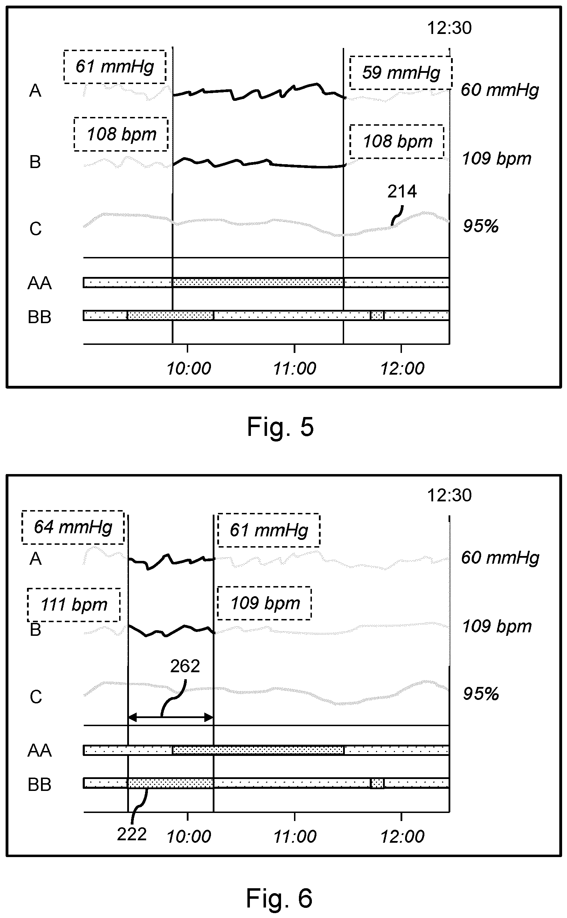

**Fig. 5** illustrates further functionality of the system of Fig. 1, in that the system may hide or deemphasize graphs of physiological parameters which are of no or lesser relevance for the selected event. For example, if the event is a medical intervention and only a subset of the physiological parameters is of particular relevance to the medical intervention, other physiological parameters may be hidden, e.g., omitted from display, or visually deemphasized. Fig. 5 shows an example of the latter, in that the system has reduced the contrast of the graph 214 pertaining to the blood oxygen saturation. This example could apply for an event being a hemodynamic intervention such as a bolus of crystalloids, which is supposed to influence mean arterial pressure, heart rate, and central venous pressure, while the influence on blood oxygen saturation is minimal or non-existent.

**Fig. 6** illustrates further functionality of the system of Fig. 1 relating to the selection of an event, in that once an event is selected by the user, the user may select a next or previous event on the common timeline, for example using left/right or up/down keys or any other similar input commands. In response to such a selection of a next or previous event, the system may adapt the time window to the selection of the next or previous event. Accordingly, the numerical visualization(s) may be updated to reflect the measured values of the physiological parameters at the start and endpoint of the newly selected event.

With continued reference to the width of the time window, the following is noted. In some embodiments, a selection of a point by the user may cause the system to establish a time window having a predetermined size centred around the selected point. For example, the time window may have a width of 4 minutes, so ±2 min relative to the selected time instance. Alternatively, the width may be selected in an adaptive manner, for example relative to other parameters, such as the total length of the timeline (e.g., the length of time for which measured values are available). For example, the width may be determined to correspond to 10% of the length of the timeline, being for example ± 24 min for 4 h of recording and ± 3 min for 0.5 h of recording, etc. As elucidated elsewhere, the width of the time window may also be determined automatically by the system, e.g., to correspond to the length of an event or to period of interest in the patient's treatment, or set manually, e.g., by the user selecting a second point in the trend view or continuing to select the first point.

In other embodiments, the user may select a point in the trend view, and the system may automatically determine the extend of the time window, for example by searching in a neighbourhood of the selected point for measured value(s) of physiological parameters which satisfy a certain condition. Examples of such conditions include, but are not limited to, measured value(s) exceeding a threshold, or being a (local) minimum or maximum, or representing a worst or best situation, or representing a certain type of physiological condition, such as a certain type of shock (e.g., a hypovolemic shock, which may be characterized by a combination of a dip in blood pressure, a concomitant dip in global end diastolic index, and an increase in systemic vascular resistance index). The system may thus analyse the measured values of the physiological parameters in a neighbourhood of the selected time instance to determine the extend of the time window.

In some embodiments, the system may have the functionality of being able to select a time window entirely automatically. For example, the system may monitor the Hemodynamic Stability Index (HSI), and when HSI is below a threshold, the system may generate a numerical visualization of the measured values of the top n physiological parameters of relevance for the HSI. These measured values may be visualized for a single time instance or for the two time instances representing endpoints of a time window.

With continued reference to the numerical visualization of measured values of physiological parameters: if there are no measured values available for a particular time instance, the system may for example display an interpolated value or a last available value or no value at all if the interpolated value or last available value is deemed inappropriate.

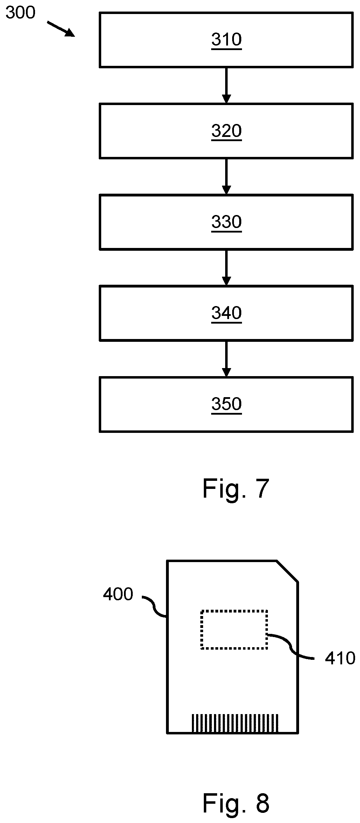

**Fig. 7** shows a block-diagram of computer-implemented method 300 for patient monitoring. The method 300 may correspond to an operation of the system 100 of Fig. 1. However, this is not a limitation, in that the computer-implemented method 300 may also be performed using another system, apparatus or device.

The method 300 is shown to comprise, in an operation titled "RECEIVING MEASUREMENT DATA", receiving 310 measurement data comprising measured values of physiological parameters of a patient at consecutive time instances. The method 300 is further shown to comprise, in an operation titled "ESTABLISHING GRAPHICAL USER INTERFACE ON DISPLAY", establishing 320 a graphical user interface on a display, wherein the graphical user interface comprises a trend view which provides a longitudinal visualization of the measurement data by setting out the measured values of the physiological parameters at the consecutive time instances against a common timeline, and in an operation titled "RECEIVING USER INPUT", receiving 330 user input representing a selection of a first point in the trend view, wherein the first point corresponds to a first time instance on the common timeline. The method 300 is further shown to comprise, in an operation titled "SELECTING TIME WINDOW", selecting 340 a time window on the common timeline based on the first time instance, and in an operation titled "DISPLAYING MEASURED VALUES AT ENDPOINTS OF TIME WINDOW", generating 350 a numerical visualization of the measured values of one or more of the physiological parameters at respective endpoints of the time window.

It will be appreciated that in general, operations of method 300 of Fig. 7 may be performed in any suitable order, e.g., consecutively, simultaneously, or a combination thereof, subject to, where applicable, a particular order being necessitated, e.g., by input/output relations. Operations may also be performed as part of other operations.

The method may be implemented on a computer as a computer implemented method, as dedicated hardware, or as a combination of both. As also illustrated in **Fig. 8****,** instructions for the computer, e.g., executable code, may be stored on a computer readable medium 400, e.g., in the form of a series 410 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 8 shows a memory device 400. Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for patient monitoring, comprising:
- an input interface (120) for receiving measurement data comprising measured values of physiological parameters of a patient at consecutive time instances;
- a user interface subsystem (180) comprising:
- a user input interface (184) to a user input device for receiving user input;
- a display output (182) to a display for displaying output of the system;
- a processor subsystem (140) configured to, via the user interface subsystem:
- establish a graphical user interface on the display, wherein the graphical user interface comprises a trend view (200) which provides a longitudinal visualization of the measurement data by setting out the measured values of the physiological parameters at the consecutive time instances against a common timeline (210);
- receive user input representing a selection of a first point (240) in the trend view, wherein the first point corresponds to a first time instance on the common timeline;
- select a time window (260, 262) on the common timeline based on the first time instance; and
- generate a numerical visualization (230-234) of the measured values of one or more of the physiological parameters at respective endpoints of the time window.

2. The system (100) according to claim 1, wherein the processor subsystem (140) is configured to receive further user input representing a selection in the trend view and to determine a width of the time window based on the further user input.

3. The system (100) according to claim 2, wherein the further user input comprises the selection (242) of a second point in the trend view, wherein the second point corresponds to a second time instance on the common timeline, and wherein the processor subsystem (140) is configured to use the first time instance as a first endpoint of the time window (260, 262) and the second time instance as a second endpoint of the time window.

4. The system (100) according to claim 2, wherein the further user input comprises a continued or repeated selection (244) of the first point, and wherein the processor subsystem (140) is configured to use the first time instance as an intermediate point, for example a midpoint, of the time window (260, 262) and to determine the width of the time window based on a time length of the continued selection, or based on a number of the repeated selection, of the first point.

5. The system (100) according to claim 1, wherein the trend view (200) further provides a visualization of an event (220, 222) with respect to the common timeline, wherein the event has a start time and an end time on the common timeline, wherein the selection of the first point is a selection of or within the visualization of the event, and wherein the processor subsystem (140) is configured to select the time window to include the event.

6. The system (100) according to claim 5, wherein the processor subsystem (140) is configured to use the start time of the event (220, 222) as a first endpoint of the time window and the end time of the event as a second endpoint of the time window.

7. The system (100) according to claim 5 or 6, wherein the event (220, 222) is associated with a subset of the physiological parameters of the patient, and wherein the processor subsystem (140) is configured to generate the numerical visualization of the measured values selectively for the subset of the physiological parameters.

8. The system (100) according to claim 7, wherein the processor subsystem (140) is configured to highlight the measured values of the subset of the physiological parameters in the trend view and/or omit or deemphasize the measured values of physiological parameters which are not part of the subset.

9. The system (100) according to any one of claims 5 to 8, wherein the processor subsystem (140) is configured to enable a user to select a next or previous event (222) on the common timeline (210) and, in response to a selection of the next or previous event by the user, adapt the time window (262) to the selection of the next or previous event.

10. The system (100) according to any one of claims 5 to 9, wherein the event (220, 222) represents or comprises a medical intervention with respect to the patient.

11. The system (100) according to any one of claims 1 to 10, wherein the processor subsystem (140) is configured to adjust a scale of the common timeline to fit the time window.

12. The system (100) according to any one of claims 1 to 11, wherein the processor subsystem (140) is configured to determine a numerical difference between the measured values of the one or more of the physiological parameters at the respective endpoints of the time window and to visualize the numerical difference.

13. The system (100) according to claim 12, wherein visualizing the numerical difference comprises visualizing a sign and/or magnitude of the numerical difference.

14. A computer-implemented method (300) for patient monitoring, comprising:
- receiving (310) measurement data comprising measured values of physiological parameters of a patient at consecutive time instances;
- establishing (320) a graphical user interface on a display, wherein the graphical user interface comprises a trend view which provides a longitudinal visualization of the measurement data by setting out the measured values of the physiological parameters at the consecutive time instances against a common timeline;
- receiving (330) user input representing a selection of a first point in the trend view, wherein the first point corresponds to a first time instance on the common timeline;
- selecting (340) a time window on the common timeline based on the first time instance; and
- generating (350) a numerical visualization of the measured values of one or more of the physiological parameters at respective endpoints of the time window.

15. A transitory or non-transitory computer-readable medium (400) comprising data (410) representing a computer program, the computer program comprising instructions for causing a processor system to perform the method according to claim 14.
